# EUROPÄISCHE PATENTSCHRIFT

(11) **EP 1 081 130 B1**
(45) Veröffentlichungstag und Bekanntmachung des Hinweises auf die Patenterteilung: **29.10.2003**
(21) Anmeldenummer: 00117137.0
(22) Anmeldetag: 10.08.2000
(51) Int. Cl.: C07C 213/10

(54) **Verfahren zur Herstellung von Alkanolaminen mit verbesserter Farbqualität**
Process for the preparation of alkanolamines with improved colour quality
Procédé de préparation d'alkanolamines avec une qualité de couleur améliorée

(30) Priorität: 04.09.1999 DE 19942300
(43) Veröffentlichungstag der Anmeldung: 07.03.2001
(73) Patentinhaber: BASF AKTIENGESELLSCHAFT, 67056 Ludwigshafen (DE)
(72) Erfinder: Ruider, Günther, Dr., 67157 Wachenheim (DE); Ross, Karl-Heinz, Dr., 67269 Grünstadt (DE); Breitscheidel, Boris, Dr., 67117 Limburgerhof (DE); Maier, Heike, 67061 Ludwigshafen (DE); Schulz, Gerhard, Dr., 67069 Ludwigshafen (DE); Huber, Sylvia, Dr., 64673 Zwingenberg (DE)

(56) Entgegenhaltungen:
- EP-A- 0 028 555
- US-A- 3 819 710
- PATENT ABSTRACTS OF JAPAN vol. 013, no. 423 (C-638), 20. September 1989 (1989-09-20) & JP 01 160947 A (MITSUBISHI GAS CHEM CO INC), 23. Juni 1989 (1989-06-23)

## Beschreibung

Die vorliegende Erfindung betrifft ein Verfahren zur Herstellung von Triethanolamin mit verbesserter Farbqualität.

Wichtige Einsatzgebiete von Triethanolamin (TEA) sind beispielsweise Seifen, Waschmittel und Shampoos in der kosmetischen Industrie oder auch Dispergiermittel und Emulgiermittel.

Für diese und andere Einsatzgebiete sind wasserklare, farblose Alkanolamine mit einer möglichst geringen Verfärbung, z. B. gemessen als APHA-Farbzahl, die diese Eigenschaften auch über längere Lagerzeiten (von z. B. 6, 12 oder mehr Monaten) beibehalten, erwünscht.

Bekannt ist, dass ein nach einer fraktionierenden Destillation eines Alkanolamin-Rohprodukts, das z. B. durch Umsetzung von Ammoniak mit Ethylenoxid oder Propylenoxid gewonnen wurde, erhaltenes und zunächst farbloses reines Alkanolamin (Farbzahl: ca. 0 bis 20 APHA nach DIN-ISO 6271(= Hazen)), sich nach einer Lagerzeit von ca. 4 bis 6 Wochen, auch im geschlossenen Gebinde und unter Lichtausschluss, allmählich leicht rosa oder leicht gelb und schließlich, besonders leicht beim Stehen am Licht, gelb bis braun verfärben kann. Dieser Effekt wird durch Einwirkung von höheren Temperaturen beschleunigt. (Siehe z.B.: G.G. Smirnova et al., J. of Applied Chemistry of the USSR 61, S. 1508-9 (1988), und Chemical & Engineering News 1996, Sept. 16, Seite 42, mittlere Spalte).

In der Literatur sind verschiedene Verfahren zur Herstellung von Alkanolaminen mit verbesserter Farbqualität beschrieben. EP-A-4015 beschreibt, dass Mono-, Di- und Triethanolamin mit geringerer Verfärbung durch Zusatz von phosphoriger oder unterphosphoriger Säure oder deren Verbindungen während oder nach der Umsetzung von Ethylenoxid mit Ammoniak und vor deren Isolierung durch Destillation erhalten werden.

EP-A-36 152 und EP-A-4015 erläutern den Einfluss der in Verfahren zur Herstellung von Alkanolaminen eingesetzten Werkstoffe auf die farbliche Qualität der Verfahrensprodukte und empfehlen nickelfreie bzw. nickelarme Stähle.

US-A-3 207 790 beschreibt ein Verfahren zur Verbesserung der Farbqualität von Alkanolaminen durch Zugabe eines Borhydrids eines Alkalimetalls in das Alkanolamin.

US-A-3 742 059 und DE-A-22 25 015 beschreiben die Verbesserung der Farbqualität von Alkanolaminen durch den Zusatz eines Alkanolaminesters der Borsäure bzw. Alkali-/Erdalkalimetallboraten.

Die Anwesenheit eines Hilfsstoffes (Stabilisators) zur Verbesserung der Farbqualität von Alkanolaminen ist jedoch in vielen wichtigen Anwendungsbereichen unerwünscht.

Die ältere deutsche Anmeldung Nr. 19855383.8 vom 01.12.98 betrifft ein Verfahren zur Reinigung von TEA, hergestellt durch Umsetzung von wässrigem Ammoniak mit Ethylenoxid in flüssiger Phase unter Druck und bei erhöhter Temperatur, indem man vom Umsetzungsprodukt überschüssiges Ammoniak, Wasser und Monoethanölamin abtrennt, das so erhaltene Rohprodukt mit Ethylenoxid bei Temperaturen von 110 bis 180 °C umsetzt und anschließend in Gegenwart von phosphoriger oder unterphosphoriger Säure oder deren Verbindungen rektifiziert.

US-A-3,819,710 offenbart ein Verfahren zur Verbesserung der Farbqualität von Ethanolaminen durch Hydrierung der rohen Ethanolamine in Gegenwart ausgewählter Katalysatoren, wie z. B. Pt, Pd, Ru oder bevorzugt Raney-Nickel. Das Verfahren führt nicht zu einer Ethanolamin-Ware, die über mehrere Monate farblos bleibt.

Erfindungsgemäß wurde weiterhin erkannt, dass es bei der Verwendung von Raney-Katalysatoren generell nachteilig ist, dass sich im Reaktionsaustrag unerwünschte Aluminiumspuren befinden, da die Alkanolamine gegenüber Aluminium als Komplexbildner wirken. Dies führt zu einer nachhaltigen Schädigung der Raney-Struktur und damit zu einer Verminderung der Stabilität und Aktivität dieser Katalysatoren.

Zudem wurde erfindungsgemäß erkannt, dass sich bei der Verwendung von Raney-Nickel oder Raney-Cobalt als Katalysator bei der Reinigung von Alkanolaminen Ni- oder Co-Spuren im Reaktionsaustrag finden, da die Alkanolamine auch gegenüber Nickel und Cobalt als Komplexbildner wirken.

EP-A-28 555 lehrt ein Verfahren zur Reinigung von N,N-Dialkylaminoethanolen durch katalytische Hydrierung in heterogener Phase und anschließende Rektifikation (vergl. Anspruch 1 und Seite 2, Zeilen 23 bis 30), wobei der Katalysator ein Metall, ausgewählt aus der VIII. Gruppe des Periodensystems, wie z. B. Ni, Co, Pt, Rh oder Pd, enthält.

JP-A-011 609 47 (Derwent Abstr. Nr. 89-224471/31, Chem. Abstr. 111: 232081r (1989)) beschreibt die Reinigung von Dialkylaminoethanol durch die Schritte (a) Entfernung hochsiedender Verunreinigungen, (b) Behandlung mit Wasserstoff in Gegenwart eines Hydrierkatalysators, der bevorzugt 0,3 bis 7 Gew.-% eines Metalls der VIII. Gruppe auf einem Träger umfasst (wie z. B. Ru/C), und (c) Destillation.

Erfindungsgemäß wurde erkannt, dass an vielen Katalysator-Trägermaterialien, wie z. B. gamma-Aluminiumoxid und Magnesiumoxid, nachteilig ist, dass Alkanolamine als Komplexbildner gegenüber dem Trägermaterial oder einzelnen Komponenten des Trägermaterials wirken, das Trägermaterial somit auslaugt und sich dadurch unerwünschte Trägerbestandteile im Reaktionsaustrag befinden.

Der vorliegenden Erfindung lag die Aufgabe zugrunde, ein alternatives, wirtschaftliches, selektives und effizientes Verfahren zur Herstellung von Triethanolamin mit verbesserter Farbqualität aufzufinden. Das Verfahren soll ermöglichen, die Verfärbung von Triethanolamin, z. B. gemessen als APHA-Farbzahl, zu vermindern und die Farbstabilität zu verbessern (unerwünschte Zunahme der Farbzahl über die Lagerzeit). Durch das Verfahren sollen keine zusätzlichen Stoffe, wie z. B. Stabilisatoren oder Spuren von Metallen oder anderen Katalysatorkomponenten, in das Triethanolamin eingebracht werden, da diese Stoffe durch Katalyse von Zersetzungsreaktionen des Alkanolamins dessen Farbstabilität häufig herabsetzen und im Produkt für bestimmte Anwendungen, z. B. im Kosmetikbereich, eine Qualitätsminderung darstellen und unerwünscht sind. D. h. im Verfahren verwendete Katalysatoren müssen leaching-stabil sein. Weiterhin soll das Verfahren aufgrund von möglichst geringen Kosten auch bei einem nur niedrigen Überdruck oder bei Normaldruck durchgeführt werden können. Schließlich soll das Verfahren den Einsatz von reindestilliertem Triethanolamin ermöglichen, wobei das Verfahrensprodukt nach der Abtrennung des Heterogenkatalysators fertig anfällt ("end-of-the-pipe") und keinen weiteren Reinigungsschritt durch Destillation oder Rektifikation mehr erfordert, da eine abschließende thermische Belastung des Verfahrensprodukts durch Destillation oder Rektifikation meist eine Verschlechterung der Farbqualität zur Folge hat.

Demgemäß wurde ein erfahren zur Herstellung von Triethanolamin mit verbesserter Farbqualität gefunden, welches dadurch gekennzeichnet ist, dass man das Triethanolamin mit Wasserstoff in Gegenwart eines Hydrierkatalysators bei erhöhter Temperatur behandelt, wobei man als Hydrierkatalysator einen Heterogenkatalysator enthaltend Re, Ru, Rh, Pd, Os, Ir, Pt und/oder Ag und alpha-Aluminiumoxid als Trägermaterial einsetzt.

Im allgemeinen werden im erfindungsgemäßen Verfahren die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch inaktiven Begleitstoffe enthalten.

Die katalytisch aktive Masse kann nach Mahlung als Pulver oder als Splitt in das Reaktionsgefäß eingebracht oder bevorzugt, nach Mahlung, Vermischung mit Formhilfsmitteln, Formung und Temperung, als Katalysatorformkörper - beispielsweise als Tabletten, Kugeln, Ringe, Extrudate (z.B. Stränge) - in den Reaktor eingebracht werden.

Die katalytisch aktive Masse des Katalysators ist als die Summe der Massen der katalytisch aktiven Bestandteile und der Trägermaterialien definiert und enthält im wesentlichen ein oder mehrere Edelmetalle oder deren Verbindungen, wie z. B. Oxide, ausgewählt aus der Gruppe Re, Ru, Rh, Pd, Os, Ir, Pt und Ag, und alpha-Aluminiumoxid (α-Al₂O₃) als Trägermaterial.

Die Summe der o.g. katalytisch aktiven Bestandteile und des o.g. Trägermaterials in der katalytisch aktiven Masse - wobei die Komponenten Re, Ru, Rh, Pd, Os, Ir, Pt und Ag als Metall in der Oxidationsstufe 0 berechnet werden - beträgt üblicherweise 80 bis 100 Gew.-%, bevorzugt 90 bis 100 Gew.-%, besonders bevorzugt 95 bis 100 Gew.-%, insbesondere größer 99 Gew.-%, beispielsweise 100 Gew.-%.

Die katalytisch aktive Masse der im erfindungsgemäßen Verfahren eingesetzten Katalysatoren enthält im allgemeinen

50 bis 99,95 Gew.-%, bevorzugt 70 bis 99,95 Gew.-%, besonders bevorzugt 80 bis 99,95 Gew.-%, ganz besonders bevorzugt 90 bis 99,95 Gew.-%, α-Al₂O₃,

0,05 bis 50 Gew.-%, bevorzugt 0,05 bis 30 Gew.-%, besonders bevorzugt 0,05 bis 20 Gew.-%, ganz besonders bevorzugt 0,05 bis 10 Gew.-%, der Edelmetalle Re, Ru, Rh, Pd, Os, Ir, Pt und/oder Ag, berechnet als Metall in der Oxidationsstufe 0, und

0 bis 20 Gew.-%, bevorzugt 0 bis 10 Gew.-%, besonders bevorzugt 0 bis 5 Gew.-%, ganz besonders bevorzugt 0 bis 1 Gew.-%, ein oder mehrere Elemente (Oxidationsstufe 0) oder deren anorganische oder organische Verbindungen, ausgewählt aus den Gruppen I A bis VI A und I B bis VII B des Periodensystems und der Gruppe Fe, Co, Ni.

Bevorzugte Katalysatoren enthalten in ihrer katalytisch aktiven Masse 50 bis 99,95 Gew.-%, bevorzugt 70 bis 99,95 Gew.-%, besonders bevorzugt 80 bis 99,95 Gew.-%, ganz besonders bevorzugt 90 bis 99,95 Gew.-%, α-Al₂O₃ und 0,05 bis 50 Gew.-%, bevorzugt 0,05 bis 30 Gew.-%, besonders bevorzugt 0,05 bis 20 Gew.-%, ganz besonders bevorzugt 0,05 bis 10 Gew.-%, Ru, Rh, Pd und/oder Pt, berechnet als Metall in der Oxidationsstufe 0.

Die katalytisch aktive Masse von besonders bevorzugten Katalysatoren besteht aus 80 bis 99,95 Gew.-%, insbesondere 90 bis 99,95 Gew.-%, α-Al₂O₃ und 0,05 bis 20 Gew.-%, insbesondere 0,05 bis 10 Gew.-%, Ru, Rh, Pd und/oder Pt, berechnet als Metall in der Oxidationsstufe 0.

Zur Herstellung der im erfindungsgemäßen Verfahren verwendeten Katalysatoren sind verschiedene Verfahren möglich.

So sind die Katalysatoren mit o.g. oxidischem Trägermaterial, die nur aus katalytisch aktiver Masse bestehen, beispielsweise durch Peptisieren pulvriger Mischungen der Hydroxide, Carbonate, Oxide und/oder anderer Salze der Katalysatorkomponenten mit Wasser und nachfolgendes Extrudieren und Tempern der so erhaltenen Masse erhältlich.

Zur Herstellung der im erfindungsgemäßen Verfahren verwendeten Katalysatoren mit oxidischem Trägermaterial, die nur aus katalytisch aktiver Masse bestehen, können Fällungsmethoden angewendet werden. So können sie beispielsweise durch eine gemeinsame Fällung der Metall-Komponenten aus einer diese Metalle enthaltenden, wässrigen Salzlösung mittels Mineralbasen in Gegenwart einer Aufschlämmung oder Suspension feinkörniger Pulver einer schwerlöslichen sauerstoffhaltigen Aluminiumverbindung und anschließendes Waschen, Trocknen und Calcinieren des erhaltenen Präzipitats erhalten werden. Als schwerlösliche, sauerstoffhaltige Aluminiumverbindung kann beispielsweise Aluminiumoxid Verwendung finden.

Vorteilhaft werden die im erfindungsgemäßen Verfahren verwendeten Katalysatoren mit oxidischem Trägermaterial, die nur aus katalytisch aktiver Masse bestehen, über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist.

Die Art der verwendeten Salze ist im allgemeinen nicht kritisch: Da es bei dieser Vorgehensweise vornehmlich auf die Wasserlöslichkeit der Salze ankommt, ist ein Kriterium ihre, zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Alpha-Aluminiumoxid ist in der Regel nicht durch Fällung direkt herstellbar, sondern entsteht erst beim späteren Calzinieren des gefällten Aluminiumoxids (gamma-Aluminiumoxid) bei Temperaturen von mindestens 900°C.

Die bei diesen Fällungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden wie üblich zu den erfindungsgemäßen Katalysatoren weiterverarbeitet. Nach dem Waschen werden sie im allgemeinen bei 80 bis 200°C, vorzugsweise bei 100 bis 150°C, getrocknet und danach calciniert. Die Calcinierung wird im allgemeinen bei Temperaturen zwischen 300 und 1100°C, vorzugsweise 400 bis 600°C, insbesondere 450 bis 550°C, ausgeführt. Für die Umwandlung von gamma-Aluminiumoxid oder Aluminiumoxiden anderer Modifikation oder Gemischen davon in alpha-Aluminiumoxid wird die Calcinierung bei Temperaturen von mindestens 900°C durchgeführt.

Nach der Calcinierung wird der Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsäure vermischt, mittels einer Presse zu Formlingen, z. B. Tabletten, verpresst und tempert. Die Tempertemperaturen entsprechen dabei im allgemeinen den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Bevorzugt werden die im erfindungsgemäßen Verfahren verwendeten Katalysatoren durch Tränkung von alpha-Aluminiumoxid (α-Al₂O₃) als Trägermaterial, das beispielsweise in Form von Pulver, Splitt oder Formkörpern, wie Strängen, Tabletten, Kugeln oder Ringen, vorliegt, hergestellt.

Die Herstellung von Formkörpern des o.g. Trägermaterials kann nach den üblichen Verfahren erfolgen.

Die Tränkung des Trägermaterials erfolgt ebenfalls nach den üblichen Verfahren, wie z. B. in EP-A-599 180, EP-A-673 918 oder A. B. Stiles, Catalyst Manufacture - Laboratory and Commercial Preparations, Marcel Dekker, Seiten 89 bis 91, New York (1983), beschrieben, durch Aufbringung einer jeweils entsprechenden Metallsalzlösung in einer oder mehreren Tränkstufen, wobei als Metallsalze z. B. entsprechende Nitrate, Acetate oder Chloride verwendet werden. Die Masse wird im Anschluss an die Tränkung getrocknet und ggf. kalziniert.

Die Tränkung kann nach der sogenannten "incipient wetness"-Methode erfolgen, bei der das oxidische Trägermaterial entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu kalzinieren. Die mehrstufige Tränkung ist vorteilhaft besonders dann anzuwenden, wenn das Trägermaterial mit einer größeren Metallmenge beaufschlagt werden soll.

Zur Aufbringung mehrerer Metallkomponenten auf das Trägermaterial kann die Tränkung gleichzeitig mit allen Metallsalzen oder in beliebiger Reihenfolge der einzelnen Metallsalze nacheinander erfolgen.

Eine Sonderform der Tränkung stellt die Sprühtrocknung dar, bei der der erwähnte Katalysatorträger in einem Sprühtrockner mit der oder den aufzubringenden Komponente(n) in einem geeigneten Lösungsmittel besprüht wird. Vorteilhaft bei dieser Variante ist die Kombination von Aufbringen und Trocknung der Aktivkomponente(n) in einem Schritt.

Die im erfindungsgemäßen Verfahren verwendeten Katalysatoren können vor ihrem Einsatz reduziert werden. Die Reduktion kann drucklos oder unter Druck erfolgen. Wenn drucklos reduziert wird, geht man so vor, dass der Katalysator unter Inertgas, beispielsweise Stickstoff, bis zu der Reduktionstemperatur erhitzt wird und dann langsam das Inertgas gegen Wasserstoff ersetzt wird.

Bei einer Reduktion unter Druck geht man praktischerweise so vor, dass man bei den später im erfindungsgemäßen Verfahren angewendeten Drücken und Temperaturen auch die Reduktion vornimmt. Je nach Temperatur und Wasserstoffdruck wird die Dauer der Reduktion gewählt, d. h. je drastischer die Bedingungen sind, desto kürzer kann die Reduktionszeit gewählt werden.

Im allgemeinen wird bei einer Temperatur von 80 bis 250°C, einem Wasserstoffdruck von 0,5 bis 350 bar und einer Dauer von 1 bis 48 h reduziert.

Es ist jedoch ebenfalls möglich, die nicht reduzierten Katalysatoren im erfindungsgemäßen Verfahren einzusetzen. In diesem Fall erfolgt die Reduktion des jeweiligen Katalysators dann gleichzeitig unter den Verfahrensbedingungen. Nach einer kurzen Betriebszeit des erfindungsgemäßen Verfahrens von einigen Stunden oder wenigen Tagen ist die Reduktion des Katalysators üblicherweise nahezu vollständig.

Beispiele für im erfindungsgemäßen Verfahren verwendbare Katalysatoren sind Trägerkatalysatoren gemäß WO 96/36589, die 0,05 bis 50 Gew.-% Silber, Ruthenium, Rhodium, Palladium, Osmium, Iridium, Platin oder deren Gemische und als Trägermaterial α-Aluminiumoxid enthalten.

Das im erfindungsgemäßen Verfahren eingesetzte Triethanolamin kann nach bekannten Verfahren, z. B. durch Umsetzung von Ammoniak mit Ethylenoxid (z. B. gemäß EP-A-673 920) erhalten werden.

Die Reinheit des im erfindungsgemäßen Verfahren eingesetzten Triethanolamins beträgt im allgemeinen größer 70 Gew.-%, insbesondere größer 80 Gew.-%. Bevorzugt wird destilliertes Triethanolamin mit einer Reinheit von ≥ 97 Gew.-%, insbesondere ≥ 98 Gew.-%, ganz besonders ≥ 99 Gew.-% eingesetzt. Es können auch Lösungen von Triethanolamin in einem inerten Lösungsmittel, wie z. B. Wasser, Alkohole (Methanol, Ethanol, iso-Propanol, n-Propanol, n-Butanol, 2-Ethylhexanol), Ammoniak, Ether (Tetrahydrofuran, Dioxan), Kohlenwasserstoffe (Toluol, Xylol, Benzol, Pentan, Hexan, Heptan, Mihagol, Petrolether), eingesetzt werden.

Die APHA-Farbzahl des eingesetzten Triethanolamins (bezogen auf das nicht säurebehandelte Triethanolamin) beträgt im allgemeinen ≤ 100, insbesondere ≤ 50, ganz besonders ≤ 20. Das erfindungsgemäße Verfahren lässt sich wie folgt ausführen:

Das verfärbte und/oder farbunstabile Triethanolamin, wird in flüssiger Phase mit Wasserstoff in Gegenwart des Hydrierkatalyators bei erhöhten Temperaturen, beispielsweise 70 bis 160°C, insbesondere 80 bis 150°C, ganz besonders 100 bis 125°C, behandelt.

Die Behandlung des Triethanolamins mit Wasserstoff kann bei Normaldruck oder unter Druck, beispielsweise bei einem Überdruck von 0 bis 50 bar (0 und 5 MPa), durchgeführt werden. Auch höhere Drücke sind möglich. Bevorzugt ist ein Überdruck von 0 bis 30 bar, insbesondere von 0 bis 20 bar.

Der Wasserstoff wird bei der Behandlung des Triethanolamins im allgemeinen in einem großen molaren Überschuss bezogen auf das Triethanolamin eingesetzt.

Die erfindungsgemäße Behandlung des Triethanolamins mit Wasserstoff in Gegenwart des Hydrierkatalysators kann sowohl kontinuierlich, beispielsweise in Rohrreaktoren in Riesel-, Sumpfoder Kreislauffahrweise, Rührbehältern oder Rührbehälterkaskaden, als auch diskontinuierlich, beispielsweise in Rührbehältern, durchgeführt werden. Der Katalysator ist bevorzugt als Festbett angeordnet, aber auch eine Suspensionsfahrweise ist möglich.

Die erforderliche Verweilzeit des Triethanolamins am Katalysator ergibt sich unter anderem aus dem Grad der Verfärbung des eingesetzten Triethanolamins und dem Ausmaß der gewünschten Entfärbung und/oder Farbstabilität des Triethanolamins. Sie ist in der Regel umso größer, je höher der Grad der Verfärbung des im erfindungsgemäßen Verfahren eingesetzten Triethanolamins ist und je höher die Anforderungen an die farbliche Qualität des Verfahrensproduktes sind.

Je nach den gewählten Reaktionsbedingungen sind im allgemeinen Verweilzeiten von 10 Minuten bis wenigen Stunden, insbesondere von 10 Minuten bis 2 Stunden, besonders von 20 bis 100 Minuten, ganz besonders von 30 bis 80 Minuten, ausreichend.

Bei einer kontinuierlichen Durchführung des Verfahrens, z. B. in einem Rohrreaktor mit Katalysatorfestbett, liegt die Katalysatorbelastung üblicherweise bei 0,5 bis 5 kg_{Triethanolamin} / (l_{Kat.} • h), bevorzugt bei 0,75 bis 3 kg_{Triethanolamin} / (l_{Kat.} • h), besonders bevorzugt bei 0,8 bis 2 kg_{Triethanolamin} / (l_{Kat.} **•** h). Die Angaben zum Volumen des Katalysators beziehen sich auf das Schüttvolumen.

Im Anschluß an die erfindungsgemäße Behandlung des Triethanolamins mit Wasserstoff in Gegenwart des Heterogenkatalysators wird der Katalysator aus dem Triethanolamin abgetrennt. Dies kann beispielsweise durch eine Dekantierung und/oder Filtration und/oder Zentrifugation erfolgen. Der zurückgewonnene Katalysator kann in der Regel wieder im Verfahren eingesetzt werden.

Ein wesentlicher Vorteil des erfindungsgemäßen Verfahrens ist, dass das erhaltene Verfahrensprodukt im wesentlichen frei von Verunreinigungen ist, die vom eingesetzten Katalysator herrühren, und somit praktisch die gleiche Reinheit wie das im Verfahren eingesetzte Triethanolamin besitzt. Das hergestellte Triethanolamin enthält im allgemeinen 0,1 bis 30 ppm, insbesondere 0,1 bis 20 ppm, ganz besonders 0,1 bis 10 ppm, an Verunreinigungen, die vom eingesetzten Katalysator herrühren, wie z. B. Re, Ru, Rh, Pd, Os, Ir, Pt, Ag und/oder Al, und wird daher in der bevorzugten Ausführungsform nach der erfindungsgemäßen Behandlung nicht weiter destillativ aufgearbeitet.

Die ppm-Angaben beziehen sich auf Gewichtsteile der Elemente in der Oxidationsstufe 0.

Das erfindungsgemäße Verfahren liefert ein in der Farbqualität verbessertes Triethanolamin, das direkt nach seinem Erhalt eine APHA-Farbzahl von 0 bis 15, insbesondere von 0 bis 10, ganz besonders von 0 bis 5, aufweist und das nach einer Säurebehandlung, die wie unten unter C.1) beschrieben innerhalb von 0,5 bis 1 Stunde nach dessen Erhalt ausgeführt wird, eine APHA-Farbzahl von 0 bis 100, insbesondere von 0 bis 80, ganz besonders von 0 bis 70, und einen Absolutwert für die Maßzahl a* nach dem CIE-Lab-System von 0 bis 3, insbesondere von 0 bis 2, ganz besonders von 0 bis 1,7, aufweist.

Das Verfahrensprodukt weist auch nach einer Lagerzeit von mindestens 4 Monaten in einem geschlossenen Gebinde unter Lichtausschluss bei Temperaturen von 10 bis 30 °C nach einer Säurebehandlung, die wie unten unter C.1) beschrieben ausgeführt wird, eine APHA-Farbzahl von 0 bis 100, insbesondere von 0 bis 80, ganz besonders von 0 bis 70, und einen Absolutwert für die Maßzahl a* nach dem CIE-Lab-System von 0 bis 3,5, insbesondere von 0 bis 3, ganz besonders von 0 bis 2,5, auf.

### Beispiele

### Allgemeine Vorbemerkungen

### A) Katalysatorpräparationen

Das in den Vergleichsbeispielen 1, 2 und 4 eingesetzte Raney-Nikkel ist handelsüblich (Fa. Degussa, Typ: B113 W) und wurde in Form der 1,5 mm Stränge eingesetzt und vor Verwendung getrocknet.

Der Pd/γ-Al₂O₃-Katalysator aus Vergleichsbeispiel 3 wurde hergestellt nach WO 96/36589 (dort Katalysator A).

### B) Apparaturen

Für die Durchführung der Versuche wurden zwei Apparaturen für den kontinuierlichen Betrieb verwendet, eine für Versuche unter Druck, die andere für Versuche unter normalem Druck (Atmosphärendruck).

### B. 1) Druckapparatur

Der Zulauf wurde über eine Kolbenpumpe zudosiert und der Wasserstoff über ein Regelventil auf den gewünschten Reaktionsdruck (bis ca. 45 bar) aufgepreßt.

Der Reaktor bestand aus Edelstahl mit Doppelmantelrohr (Ölheizung) und hatte ein lichtes Volumen von 40 ml, das für die Katalysatorfüllung zur Verfügung steht. Reaktoreingang und -ausgang wurden mit Glaskugeln versetzt, um ein Entweichen des Katalysators zu verhindern. Im Abgas des Reaktors wurde der Volumenstrom gemessen, der Abgasstrom während der Behandlung wurde generell auf 5 l H₂/h eingestellt. Die Standhaltung erfolgte über ein Ventil, das über eine Differenzdruckmessung im Abscheider gesteuert wurde.

### B.2) Normaldruckapparatur

Der Zulauf wurde über eine Kolbenpumpe dosiert und Wasserstoff über ein Regelventil auf normalen Druck entspannt; die Messung des Wasserstoffflusses erfolgte über eine Massenstrommessung auf der Zulaufseite und wurde auf einen Wert von 5 l H₂/h eingestellt. Der Reaktor bestand aus Glas mit Doppelmantelrohr (Ölheizung) und hatte ein beheiztes Volumen von 70 ml. Am Reaktoreingang und - ausgang wurden jeweils Glaskugeln plaziert, so dass für die Katalysatorschüttung etwa ein Volumen von 30 ml verblieb. Der Reaktoraustrag wurde über einen Luftkühler gekühlt und in ein Austragsgefäss gefördert.

### C) Bestimmung der Farbqualität und Farbstabilität des Alkanolamins

Zur Bestimmung der Farbqualität und Farbstabilität der behandelten Alkanolamine wurden von den wie unten beschrieben behandelten Alkanolaminen (a) direkt nach der Behandlung und (b) nach Lagerung bei Raumtemperatur unter Luftatmosphäre in geschlossenen Gefäßen unter Lichtausschluß nach den in den Tabellen Nr. 1 und 2 angegebenen Lagerzeiten Proben gezogen. Diese Proben wurden zunächst der unten beschriebenen Säurebehandlung unterworfen, um auftretende Farbeffekte zu verstärken, und direkt anschließend wurden in einer spektralen Farbmessung die Werte für die Maßzahlen a* und b* nach dem CIE-Lab-System (nach Judd und Hunter (CIE = Comission International d'Eclairage, Paris); (vergl. DIN 6174)) und der APHA-Wert (entsprechend DIN-ISO 6271) bestimmt. Die Bestimmung der a*-, b*- und APHA-Werte (APHA- = Hazen- = Pt/Co-Farbzahl) erfolgte standardmäßig in einem LICO 200-Gerät der Firma Dr. Lange in einer 5 cm Küvette (Volumen ≈ 20 ml). Der a*-Wert gibt die Rot-/Grünfärbung der Probe an (ein positiver a*-Wert gibt den roten Farbanteil an, ein negativer a*-Wert den grünen Farbanteil) und der b*-Wert den Gelb-/Blauanteil (ein positiver b*-Wert gibt den gelben Farbanteil an, ein negativer b*-Wert den blauen Farbanteil). Anzustreben ist besonders ein geringerer absoluter a*-Wert als im Ausgangsmaterial vor der Behandlung.

Die in den Tabellen Nr. 1 und 2 angegebenen a*-, b*- und APHA-Werte beziehen sich stets auf die Proben nach der durchgeführten Säurebehandlung.

### C.1) Säurebehandlung des Alkanolamins

[Eine Säurebehandlung eines Alkanolamins zur Verstärkung von Farbeffekten wurde allgemein in JP-A-62 019 558 (Derwent Abstract Nr. 87-067647/10) und JP-A-62 005 939 (Derwent Abstract Nr. 87-047397/07) beschrieben, wonach TEA mit Essigsäure, Zitronensäure, Schwefelsäure, Salzsäure oder Phosphorsäure behandelt (neutralisiert) wird.]

Die Säurebehandlung wurde, wenn nicht anders angegeben, wie folgt ausgeführt:

Das Alkanolamin wurde mit 1000 ppm (Gewichtsteile) Eisessig vermischt und unter Stickstoff 3 h auf 100 °C erhitzt.

### D) Analytik und Allgemeines

Spuren von Metallen im Alkanolamin nach der Behandlung in Gegenwart des Katalysators wurden über Atomabsorptionsspektroskopie ermittelt. Die Angabe in den Tabellen erfolgt in ppm (= mg/kg).

Die Belastung des Katalysators wird in den Tabellen in 1_{Alkanolamin} / (1_{Katalysator} • h) angegeben.

Das in den Beispielen eingesetzte Triethanolamin besaß eine Reinheit nach GC von ≥ 99 Fl.-% und wies nach der oben unter D.1) beschriebenen Säurebehandlung generell a*- und b*-Werte zwischen 3 und 4 auf, die APHA-Farbzahl lag zwischen 30 und 50.

Das in den Beispielen eingesetzte Triethanolamin wies ohne vorherige Säurebehandlung einen a*-Wert von 0,2, einen b*-Wert von 1,0 und eine APHA-Farbzahl von 10 auf.

Die Drücke und Temperaturen, bei denen die jeweiligen Beispiele durchgeführt wurden, sind in den Tabellen Nr. 1 und 2 angegeben.

Die Ergebnisse der Behandlung des Alkanolamins mit Wasserstoff bei einem Druck oberhalb von Normaldruck sind in den Vergleichsbeispielen 1 bis 3 und 5 sowie in den Beispielen 1 bis 21 (vgl. Tabelle 1) dargestellt.

Die Ergebnisse der Behandlung des Alkanolamins mit Wasserstoff bei normalem Druck (ND) sind in dem Vergleichsbeispiel 4 sowie in den Beispielen 22 bis 35 (vgl. Tabelle 2) dargestellt.

### Vergleichsbeispiele 1 und 2

30 ml Raney-Nickel (1,5 mm Stränge) wurden bei 150°C im Stickstoffstrom (10 l/h) getrocknet. Triethanolamin wurde bei dem angegebenen Druck und der angegebenen Temperatur bei einem Wasserstoffstrom von 5 l H₂/h an dem so aktivierten Katalysator behandelt.

Zwar ergaben die Behandlungen in Gegenwart von Raney-Nickel eine akzeptable Absenkung der a*-Werte, jedoch lag der b*-Wert zum Teil über dem des Ausgangsproduktes.

Zudem fanden sich im Austrag die angegebenen Nickel- und insbesondere Aluminiumspuren, was unakzeptabel ist.

### Vergleichsbeispiel 3

30 ml des Katalysators mit der Zusammensetzung 0,58 Gew.-% Pd auf γ-Al₂O₃ wurde 16 h bei 180°C unter einem H₂-Strom von 10 l/h aktiviert. An diesem so aktivierten Katalysator wurde Triethanolamin bei dem angegebenen Druck und der angegebenen Temperatur bei einem Wasserstoffstrom von 5 1 H₂/h behandelt.

Der Reaktionsaustrag war bereits mit bloßem Auge sichtbar rosa gefärbt, sowohl der a*- als auch der b*-Wert waren deutlich erhöht und Aluminium wurde ausgelaugt.

### Vergleichsbeispiel 4

20 ml Raney-Nickel wurde 3 h bei 150°C unter einem H₂-Strom von 5 l/h getrocknet. An diesem so aktivierten Katalysator wurde Triethanolamin bei normalem Druck und der angegebenen Temperatur bei einem Wasserstoffstrom von 5 l H₂/h behandelt.

Im Austrag fanden sich Aluminium und Nickel.

### Vergleichsbeispiel 5

33 ml des Katalysators mit der Zusammensetzung 1 Gew.-% Ru auf Aktivkohle wurden 3 h bei 100°C in einem Strom von 10 l H₂/h aktiviert.

Der Austrag war noch nach 65 Betriebsstunden getrübt, der Ansatz wurde verworfen.

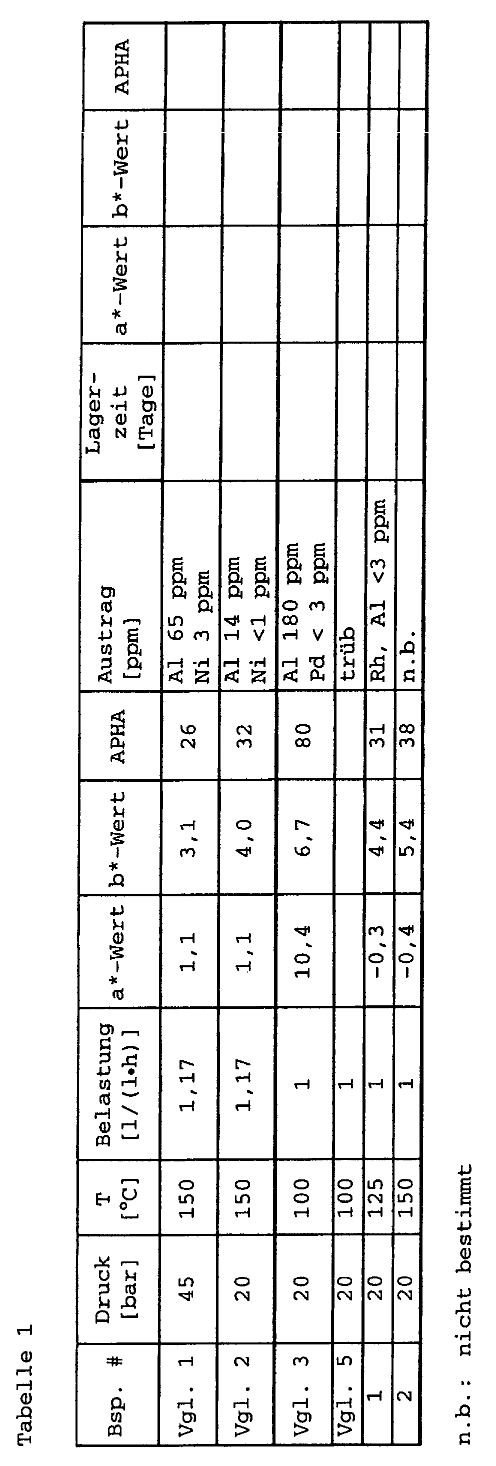

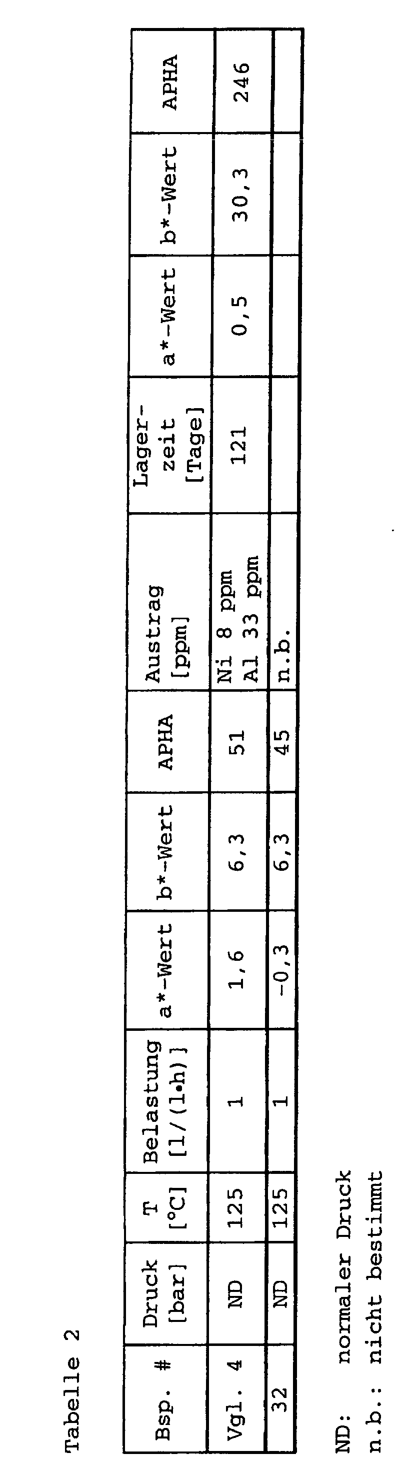

## Patentansprüche

1. Verfahren zur Herstellung von Triethanolamin mit verbesserter Farbqualität, **dadurch gekennzeichnet, dass** man das Triethanolamin mit Wasserstoff in Gegenwart eines Hydrierkatalysators bei erhöhter Temperatur behandelt, wobei man als Hydrierkatalysator einen Heterogenkatalysator enthaltend Re, Ru, Rh, Pd, Os, Ir, Pt und/oder Ag und alpha-Aluminiumoxid als Trägermaterial einsetzt.

2. Verfahren nach Anspruch 1, **dadurch gekennzeichnet, dass** man einen Hydrierkatalysator enthaltend Ru, Rh, Pd und/oder Pt einsetzt.

3. Verfahren nach den Ansprüchen 1 bis 2, **dadurch gekennzeichnet, dass** die katalytisch aktive Masse des Katalysators 50 bis 99,95 Gew.-% des Trägermaterials und 0,05 bis 50 Gew.-% des Edelmetalls, berechnet als Metall in der Oxidationsstufe 0, enthält.

4. Verfahren nach Ansprüchen 1 bis 2, **dadurch gekennzeichnet, dass** die katalytisch aktive Masse des Katalysators 70 bis 99,95 Gew.-% des Trägermaterials und 0,05 bis 30 Gew.-% des Edelmetalls, berechnet als Metall in der Oxidationsstufe 0, enthält.

5. Verfahren nach Ansprüchen 1 bis 2, **dadurch gekennzeichnet, dass** die katalytisch aktive Masse des Katalysators aus 80 bis 99,95 Gew.-% des Trägermaterials und 0,05 bis 20 Gew.-% des Edelmetalls, berechnet als Metall in der Oxidationsstufe 0, besteht.

6. Verfahren nach den Ansprüchen 1 bis 5, **dadurch gekennzeichnet, dass** man die Behandlung bei einem Überdruck von 0 bis 50 bar durchführt.

7. Verfahren nach den Ansprüchen 1 bis 6, **dadurch gekennzeichnet, dass** man die Behandlung bei einer Temperatur von 70 bis 160°C durchführt.

8. Verfahren nach den Ansprüchen 1 bis 7 zur Herstellung von Triethanolamin, das während einer viermonatigen Lagerung in einem geschlossenen Gebinde unter Lichtausschluss bei Temperaturen von 10 bis 30 °C nach einer Säurebehandlung eine APHA-Farbzahl (DIN-ISO 6271) von 0 bis 100 und einen Absolutwert für die Maßzahl a* nach dem CIE-Lab-System von 0 bis 3,5 aufweist.

9. Verfahren nach den Ansprüchen 1 bis 8 zur Herstellung von Triethanolamin, das 0,1 bis 30 ppm (Gewichtsteile) an Verunreinigungen, die vom Hydrierkatalysator herrühren, aufweist.

## Claims

1. A process for the preparation of triethanolamine having improved color quality by treating the alkanolamine with hydrogen in the presence of a hydrogenation catalyst at elevated temperature, which comprises using, as hydrogenation catalyst, a heterogeneous catalyst comprising Re, Ru, Rh, Pd, Os, Ir, Pt and/or Ag and alpha-aluminum oxide as support material.

2. A process as claimed in claim 1, which comprises using a hydrogenation catalyst comprising Ru, Rh, Pd and/or Pt.

3. A process as claimed in claims 1 to 2, wherein the catalytically active mass of the catalyst comprises from 50 to 99.95% by weight of the support material and from 0.05 to 50% by weight of the noble metal, calculated as metal in oxidation state 0.

4. A process as claimed in claims 1 to 2, wherein the catalytically active mass of the catalyst comprises from 70 to 99.95% by weight of the support material and from 0.05 to 30% by weight of the noble metal, calculated as metal in oxidation state 0.

5. A process as claimed in claims 1 to 2, wherein the catalytically active mass of the catalyst consists of from 80 to 99.95% by weight of the support material and from 0.05 to 20% by weight of the noble metal, calculated as metal in oxidation state 0.

6. A process as claimed in claims 1 to 5, wherein the treatment is carried out at a superatmospheric pressure of from 0 to 50 bar.

7. A process as claimed in claims 1 to 6, wherein the treatment is carried out at a temperature of from 70 to 160°C.

8. A process as claimed in claims 1 to 7, wherein the prepared triethanolamine, during storage for four months in a sealed container with the exclusion of light at temperatures of from 10 to 30°C, has, following acid treatment, an APHA color number (DIN-ISO 6271) of from 0 to 100 and an absolute value for the numerical measure a* according to the CIE-Lab system of from 0 to 3.5.

9. A process as claimed in claims 1 to 8, wherein the prepared triethanolamine has from 0.1 to 30 ppm (parts by weight) of impurities which originate from the hydrogenation catalyst.

## Revendications

1. Procédé de préparation de triéthanolamine ayant une qualité de couleur améliorée, **caractérisé en ce qu'**on traite la triéthanolamine avec de l'hydrogène en présence d'un catalyseur d'hydrogénation à haute température, le catalyseur d'hydrogénation utilisé étant un catalyseur hétérogène contenant du Re, du Ru, du Rh, du Pd, de l'Os, de l'Ir, du Pt et/ou de l'Ag, et de l'oxyde d'aluminium alpha en tant que matériau support.

2. Procédé selon la revendication 1, **caractérisé en ce qu'**on utilise un catalyseur d'hydrogénation contenant du Ru, du Rh, du Pd et/ou du Pt.

3. Procédé selon les revendications 1 à 2, **caractérisé en ce que** la masse catalytiquement active du catalyseur contient de 50 à 99,95 % en poids du matériau support et de 0,05 à 50 % en poids du métal précieux, calculé en métal au degré d'oxydation 0.

4. Procédé selon les revendications 1 à 2, **caractérisé en ce que** la masse catalytiquement active du catalyseur contient de 70 à 99,95 % en poids du matériau support et de 0,05 à 30 % en poids du métal précieux, calculé en métal au degré d'oxydation 0.

5. Procédé selon les revendications 1 à 2, **caractérisé en ce que** la masse catalytiquement active du catalyseur est constituée de 80 à 99,95 % en poids du matériau support et de 0,05 à 20 % en poids du métal précieux, calculé en métal au degré d'oxydation 0.

6. Procédé selon les revendications 1 à 5, **caractérisé en ce qu'**on met en oeuvre le traitement sous une surpression de 0 à 50 bar.

7. Procédé selon les revendications 1 à 6, **caractérisé en ce qu'**on met en oeuvre le traitement à une température de 70 à 160°C.

8. Procédé selon les revendications 1 à 7 pour préparer de la triéthanolamine, qui pendant un stockage de quatre mois dans un récipient fermé, à l'abri des la lumière et à une température de 10 à 30°C, présente après un traitement par un acide un indice de coloration APHA (DIN-ISO 6271) de 0 à 100 et une valeur absolue pour la composante a* selon le système CIE-Lab de 0 à 3,5.

9. Procédé selon les revendications 1 à 8 pour préparer une triéthanolamine qui contient de 0,1 à 30 ppm (parties en poids) d'impuretés provenant du catalyseur d'hydrogénation.
